# EUROPEAN PATENT APPLICATION

(11) **EP 3 653 734 A1**
(43) Date of publication of application: **20.05.2020**
(21) Application number: 19182694.0
(22) Date of filing: 26.06.2019
(51) Int. Cl.: C12Q 1/6888

(54) **NEW METHODS FOR SPECIES IDENTIFICATION**

(30) Priority: 16.11.2018 EP 18206755
(71) Applicant: Ares Trading S.A., 1170 Aubonne (CH)
(72) Inventor: Bachmann, Alessia, 10010 Colleretto Giacosa TO (IT); La Neve, Fabio, 10010 Colleretto Giacosa TO (IT)
(74) Representative: Merck Serono S.A. Intellectual Property

(57) **Abstract**

Provides herein is a method for identifying the specific cell lineage of cells in culture comprising the steps of determining from the nucleic acid molecules isolated from said recombinant cells in culture the presence of polymorphisms or SNPs at at least 5 different positions within at least five genes contained in said nucleic acid molecules, obtaining a genetic profile from the determination of the previous step, and identiyfing the cell lineage of said cells in culture from said genetic profile, and wherein the recombinant cells produce a recombinant protein.

SEQUENCE LISTING

<110>  Ares Trading S.A.

<120>  New Methods for Species Identication

<130>  P 18-247

<140>  EP 18206755.3
<141>  2018-11-16

<160>  10

<170>  PatentIn version 3.5

<210>  1
<211>  20
<212>  DNA
<213>  Mammalian

<400>  1
tggagtctgt gcaagccctg                                                   20


<210>  2
<211>  25
<212>  DNA
<213>  Mammalian

<400>  2
actcaccatc aatgttgagc atcat                                             25


<210>  3
<211>  25
<212>  DNA
<213>  Mammalian

<400>  3
ttattaacca ckcattcatt gayyt                                             25


<210>  4
<211>  24
<212>  DNA
<213>  Mammalian

<400>  4
gcgaatagga aytatcattc mggt                                              24


<210>  5
<211>  20
<212>  DNA
<213>  Mammalian

<400>  5
cgggttaaag ttgatgggcc                                                   20


<210>  6
<211>  23
<212>  DNA

<213>  Mammalian

<400>  6
actgccaatt tcatctgtga caa                                              23


<210>  7
<211>  27
<212>  DNA
<213>  Mammalian

<400>  7
agtraaagtr gaatttgatg aagaatt                                          27


<210>  8
<211>  27
<212>  DNA
<213>  Mammalian

<400>  8
agcaaagaat ctcttgggat aaccaca                                          27


<210>  9
<211>  22
<212>  DNA
<213>  Mammalian

<400>  9
ckggaccttc sgtgtcggag ct                                               22


<210>  10
<211>  23
<212>  DNA
<213>  Mammalian

<400>  10
ctctkaaaag agccgttggg tta                                              23

## Description

### FIELD OF THE INVENTION

The present invention relates to biologic systems and more specifically to the use of genomic and computational analysis for bioproduction of biological molecules. In particular it relates to methods for the identification of specific cell lineage and cell bank characterization, SNPs as well as primers for amplification useful in said methods.

### BACKGROUND OF THE INVENTION

The production of recombinant therapeutic is more and more important to the pharmaceutical industry. Chinese hamster ovary (CHO) cell lines are part of the most used cells for producing recombinant proteins. Other well-known and commonly used cell lines in pharmaceutical industry are for instance NS0 or SP2/0. These cells have been repeatedly approved by regulatory agencies. They can be easily cultured in suspension and can produce high titers of human-compatible therapeutic proteins.

Health Authorities, not only before, but also after approval of a drug produced recombinantly via cells in culture require an identity test for the confirmation of the mammalian host cell. Identity tests are needed for instance to show that the cell banks that are used are stable over time and that there are no cross-contaminations. Traditional tests were based on isoenzyme analysis, which can show specie-specific mobility patterns on an electrophoresis gel. These tests are based on the difference in electrophoretic mobility of four different isoenzymes, which allows distinction to be made between human, murine and hamster species. However these enzymatic tests require reagents that may become scarce and are cumbersome to carry out. Other drawbacks include that the sensitivity of these test is not sufficient to comply with the most current acceptable standards.

Therefore, there is a need for alternative and effective methods for the identification of specific cell lineages and cell bank characterization.

### SUMMARY OF THE INVENTION

In a first aspect the present invention discloses a method for identifying the specific cell lineage of cells in culture comprising the steps of: 1) determining from the nucleic acid molecules isolated from said recombinant cells in culture the presence of polymorphisms or SNPs at at least 5 different positions, more advantageously at at least 10 different positions, even more advantageously at at least 20 different positions, within at least five genes contained in said nucleic acid molecules, 2) obtaining a genetic profile from the determination of step 1), and 3) identiyfing the cell lineage of said cells in culture from said genetic profile; wherein the at least five genes are: Argonaute RISC catalytic component 1 (Ago1), Cytochrome b (Cytb), Histone deacetylase 1 (Hdac1), Serine/arginine-rich splicing factor 1 (Srsf1) and Topoisomerase II beta (Top2b), and wherein the recombinant cells produce a recombinant protein.

In a second aspect of the invention, herein described is an analytic method comprising the steps of: 1) analyzing the nucleic acid molecules isolated from recombinant cells in culture to determine the presence of polymorphisms or SNPs at at least 5 different positions, more advantageously at at least 10 different positions, even more advantageously at at least 20 different positions, within at least five genes contained in said nucleic acid molecules, 2) obtaining a genetic profile from the analysis of step 1), and 3) determining the species of said recombinant cells in culture from said genetic profile; wherein the at least five genes are: Argonaute RISC catalytic component 1 (Ago1), Cytochrome b (Cytb), Histone deacetylase 1 (Hdac1), Serine/arginine-rich splicing factor 1 (Srsf1) and Topoisomerase II beta (Top2b), and wherein the recombinant cells produce a recombinant protein.

In a third aspect, the present invention relates to a method for cell bank characterisation of recombinant cells in culture comprising the steps of: 1) determining, in nucleic acid molecules isolated from said recombinant cells in culture, the presence of polymorphisms or SNPs at at least 5 different positions, more advantageously at at least 10 different positions, even more advantageously at at least 20 different positions, within at least five genes, 2) obtaining a genetic profile from the detection of step 1), and 3) characterizing the origine of the cell bank of the recombinant cells in culture from said genetic profile; wherein the at least five genes are: Argonaute RISC catalytic component 1 (Ago1), Cytochrome b (Cytb), Histone deacetylase 1 (Hdac1), Serine/arginine-rich splicing factor 1 (Srsf1) and Topoisomerase II beta (Top2b), and wherein the recombinant cells produce a recombinant protein.

### DETAILED DESCRIPTION OF THE INVENTION

Describer herein is a combination of single species-specific nucleotides (SNPs) allowing identification of the specific cell lineage and cell bank characterisation of cells. Thanks to these combinations of SNPs, it is possible to easily differentiate between species (e.g. Mouse, CHO, Human, etc).

In summary, the method is based on the analysis of differences between animal species (such as mammalian species) in SNPs found in the sequences of 5 highly preserved genes, using PCR (Polymerase Chain Reaction) and sequencing methods. The differences in the SNPs allow the creation of a species-specific pattern that is analyzed by bioinformatics software to confirm the cell line identity and detect any contamination by cell lines of other species. Various advantages of the methods are the following: 1) No reagents for Isoenzyme analysis, 2) Robustness (5, 10, or 20 SNPs only on 5 different genes are needed; «Forensic-like» approach), 3) Sensitivity, 4) Identification of potential contamination and 5) Cost efficient.

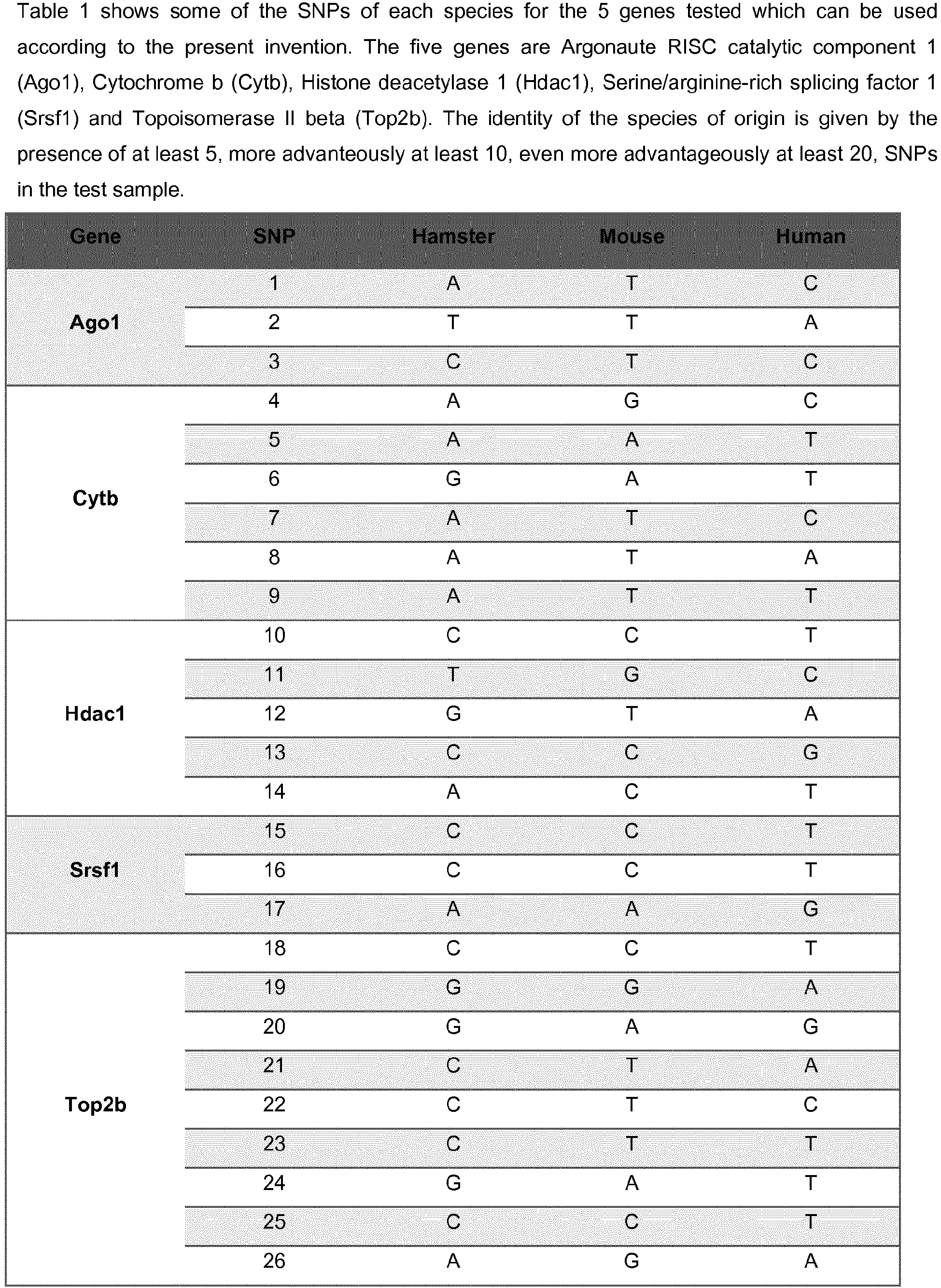

According to the present invention as a whole, the preferred at least 20 SNPs are selected from any combination of the SNP's as described in table 1. Indeed it was shown by the inventors that using these SNPs allowed for a very accurate identification/characetrization of a cell line / cell lineage.

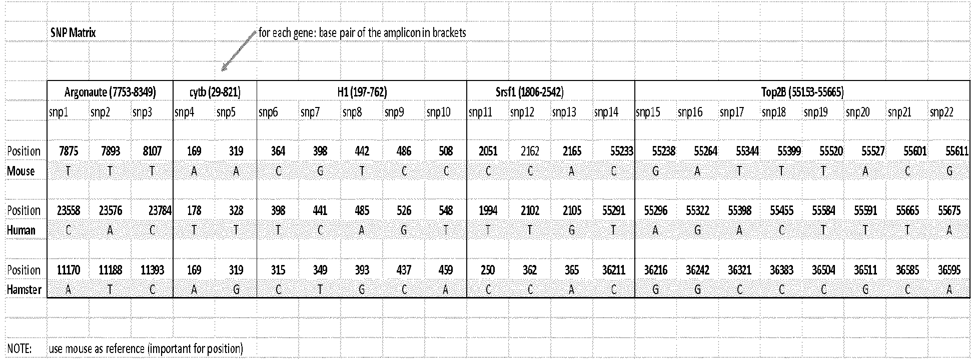

NGS = for instance PyroSequencing, Solexa-Illumina, Solid or Ion Torrent or Oxford Nanopore

### EXAMPLES

### Introduction:

Summary of the method: The method involves culturing the cell line to be analysed (sample) and preparing cell pellets. The genomic DNA extracted from the sample undergoes 5 different PCR reactions using a pair of primers specific for each of the five genes of interest. These primers amplify the region of the gene in which the SNPs are located. Subsequently, starting from purified PCR products, libraries are prepared for loading on the MiSeq (Illumina) sequencer for sequencing. The data produced are then analyzed using a specific bioinformatics pipeline, which allows the sample cell line of origin to be identified, as well as the presence of cell lines of any other species.

This method can be used to analyze (non-limiting examples):
- cell banks used to produce recombinant proteins
- cell lines used in Viral Safety testing
- cell lines used to propagate viruses and used as test systems in Viral Clearance Validation studies.

### Cell lines and antibodies:

- CHO-K1 (Chinese Hamster Ovary cell line), ATCC CCL-61,
- MRC-5 (Human Lung Fibroblast), ATCC CCL-171,
- Sp2/0-Ag14 (Mouse, Non-secreting Hybridoma), ATCC CRL-1581,
- mAb1 cell, expressing ,Ab1, based on CHO-S,
- mAb2 cell, expressing ,Ab1, based on Sp2/0-Ag14.

### Main material / kits

- QiaAmp DNA Blood kit (Qiagen)
- RNase, DNase-free (Roche)
- dNTPs (Life Technologies or equivalent)
- GeneAmp High Fidelity PCR System (Life Technologies)
- Primers (Life Technologies or equivalent): see table 2
- MinElute PCR Purification Kit (QIAGEN)
- Nextera XT Sample Preparation Kit - Box 1 and Box 2 (Illumina)
- Nextera XT Index kit (Illumina)
- Qubit dsDNA HS assay kit (Life technologies)
- Agilent High Sensitivity DNA Kit (Agilent)
- PhiX Control v3 (Illumina)
- MiSeq Reagent Nano Kit v2 (300 Cycles) (Illumina) consisting of:
   • - MiSeq v2 Reagent Kit 300 Cycles - Box 1 of 2
   • - MiSeq Reagent Nano Kit v2 - Box 2 of 2
- MiSeq Reagent Micro Kit v2 (300 Cycles) (Illumina) consisting of:
   • - MiSeq v2 Reagent Kit 300 Cycles - Box 1 of 2
   • - MiSeq Reagent Kit Micro v2 - Box 2 of 2

**Table 2, Primers**

| Gene | Forward primer sequence | Reverse primer sequence |
|---|---|---|
| Ago1 | TGGAGTCTGTGCAAGCCCTG | ACTCACCATCAATGTTGAGCATCAT |
| | (SEQ ID NO.1:) | (SEQ ID NO.2:) |
| Cytb | TTATTAACCACKCATTCATTGAYYT | GCGAATAGGAAYTATCATTCMGGT |
| | (SEQ ID NO.3:) | (SEQ ID NO.4:) |
| Srsf1 | CGGGTT AAAGTTGA TGGGCC | ACTGCCAATTTCATCTGTGACAA |
| | (SEQ ID NO.5:) | (SEQ ID NO.6:) |
| Top2b | AGTRAAAGTRGAATTTGATGAAGAATT | AGCAAAGAATCTCTTGGGATAACCACA |
| | (SEQ ID NO.7:) | (SEQ ID NO.8:) |
| Hdac1 | CKGGACCTTCSGTGTCGGAGCT | CTCTKAAAAGAGCCGTTGGGTTA |
| | (SEQ ID NO.9:) | (SEQ ID NO.10:) |

### Main methods

### Cell pellet preparation

The cells to be analysed were isolated from various test cultures and pelleted.

*Pellet production from cells in suspension:* The cells were resuspended in culture medium and then centrifuged for 10 minutes at 1000 rpm at +4°C. The supernatant was then removed. The resulting pellet can be stored at -80°C for 5 years maximum from preparation should it be needed.

*Pellet production from adherent cells*: when the cell monolayer reaches confluence, the culture medium is aspirated from the flask using a sterile pipette. Then the monolayer is washed with PBS. After removal of PBS, trypsin is distribute it evenly over the monolayer by gently moving the flask several times (e.g. 12-15 times). Once the cell monolayer has completely detached, the cells are resuspended in culture medium to block the effect of the trypsin. The cells are then centrifuged for 10 minutes at 1000 rpm at +4°C. The supernatant is then removed, The resulting pellet can be stored at -80°C for 5 years maximum from preparation should it be needed.

### Cell viability:

Before proceeding with cell pellet preparation, the following acceptance criterion were checked: cell viability ≥ 80%. If cell viability was < 80% but between 50% and 79%, cell culturing in flasks was continued until cell viability increases. If cell viability was less than 50%, the cells in culture were discarded.

### Genomic DNA extraction and quantitation

Genomic DNA was extracted using the Qiagen QiaAmp DNA Blood kit according to the instructions provided in the kit. Once extraction done, DNA was qantified using the NanoDrop method. Two measurements were made for each sample and the final concentration was the mean result. Quantitation on the NanoDrop enabled the degree of purity of each sample to be verified by assessing the 260/280 ratio. To be used in subsequent test phases, the genomic DNA from a sample should respect the following: the 260/280 ratio must be within a 1.7 - 2.1 range (inclusive). If a sample did not meet the acceptance criterion, it could not be used in subsequent test phases and genomic DNA extraction was repeated only once.

### DNA amplification

The genomic DNA extracted from samples underwent 5 different PCR reactions using a specific pair of primers for each of the 5 genes (Ago1, Cytb, Hdac1, Srsf1 and Top2b). Standard methods were used for such amplifications. It is noted that if lyophilized, the primers were resuspended in ultrapure water. Each PCR reaction included a negative amplification control consisting of PCR mix with water instead of genomic DNA. In addition, two replicates of the reaction were prepared for each sample.

The reagents in the amplification mix were used at the final concentrations as below:
- 10X PCR buffer (from GeneAmp High Fidelity PCR System kit) 1X
- dNTPs 0.2 mM
- Forward Primer 0.5 µM (see table XX)
- Reverse Primer 0.5 µM (see table XX)
- Taq Polymerase (from GeneAmp High Fidelity PCR System kit) 2 Units
- Ultrapure water Qs* (* Ultrapure water quantum sufficit, taking into account the volume of genomic DNA to be added to achieve the final volume of 40 µL (when using the Applied Biosystems Veriti Thermal Cycler) or 50 µL (when using the PE GeneAmp PCR System 9700 thermal cycler).

The amplification reaction was checked by an agarose gel electrophoretic run (prepared according to standard procedures). To go on to the subsequent PCR amplification product purification phase, the following acceptance criteria were checked:
- no bands in the negative amplification control
- presence of the expected band in both replicates of the same sample with the following molecular
- weight acceptance criteria range: expected molecular weight ± 10%

The molecular weight (bp) of the expected band for each gene in each species is shown in the table below:

| Species | Ago1 | Cytb | Hdac1 | Srsf1 | Top2b |
|---|---|---|---|---|---|
| Human | 590 | 794 | 579 | 701 | 496 |
| Hamster | 589 | 793 | 583 | 707 | 496 |
| Mouse | 597 | 793 | 577 | 707 | 490 |

If the results meet the acceptance criteria, the PCR amplification products can be purified. If the sample PCR reaction acceptance criteria are not met for a certain gene, the PCR reaction is to be repeted for that sample for that gene and the electrophoretic run only once.

### Purification and quantitation of PCR products

After checking the PCR reaction on agarose gel, the remaining volumes of amplification product were purified using the Qiagen MinElute PCR Purification Kit as instructed in the instructions provided with the kit. The PCR purification products were subsequently quantified using the Qubit dsDNA HS Assay Kit.

In order to go on to the subsequent test phases, the following acceptance criterion was checked: the lowest concentration of PCR purification product obtained for each sample must be ≥ 1 ng/µL. If the minimum sample purification product concentration was not achieved for a certain gene, the PCR reaction for that sample for that gene, the electrophoretic run and purification were repeated.

### Library preparation

The sample library was prepared by mixing the PCR purification products of the 5 genes of the sample. Libraries belonging to different samples can be loaded on a single flow cell and analyzed as a single pool for sequencing. The Illumina Experiment Manager (IEM) software installed on the MiSeq system was used to confirm the validity of the choice of the SNPs, i.e. those nucleotide sequences that allowed each sample to be univocally identified. Once the SNPs chosen, the libraries were prepared using the Illumina Nextera XT kit following the instructions provided with the kit. For each sample, the PCR purification product of each of the 5 genes was taken to a concentration of 1 ng/µL based on the quantitation from the Qubit measurement.

Before proceeding with the subsequent phases, the quality of the libraries produced was assessed. This was performed in two steps: analysis on a Qubit fluorometer (to determine the concentration) and capillary electrophoresis analysis on a Bioanalyzer (to determine the concentration and size). In both case standard procedures were used. Subsequently, 1µl of library was analyzed using the Agilent High Sensitivity DNA Kit as instructed in the kit.

To be used in subsequent test phases, the following acceptance criteria were checked for each sample library:
- Mean size of library fragments ≥ 200 bp
- Mean library concentration ≥ 2 nM
If the acceptance criteria were met, the flow cell was loaded and run on the MiSeq sequencer. If the library produced for a certain sample did not respect these criteria, library preparation was repeated.

### Loading on the MiSeq sequencer

The libraries produced were loaded either on a *Nano* or on a *Micro* flow cell. The flow cell was chosen depending on the number of samples to be tested. A *Nano* flow cell was used for 2 samples, while for a *Micro* flow cell the number of samples was 10. Libraries were loaded and run as instructed in the MiSeq sequencer

The MiSeq sequencer run parameters respected the following acceptance criteria:
- Density: 600 and 1900 K/mm2;
- %Q30: ≥ 65%;
- Cluster PF: ≥ 70%;
- Phasing and Prephasing: < 0.3

If any of the parameters were out of these acceptance criteria, he sequencing run was repeated.

### Bioinformatics data analysis

The data produced by the MiSeq sequencer run were analyzed using the MAGNETO bioinformatics pipeline.

In the genomic sequences produced by the samples, the bioinformatics pipeline seeks the profile of 22 SNPs distributed over the 5 genes listed in Table 2. At the end of the analysis, the pipeline produced a report containing one or more tables depending on the species-specific profiles identified. This allowed confirmation of the species of origin of the test cell line and assessment of any cross-contamination with cells of other species.

### Assessment of results - Test validity

The test was considered valid if the following conditions occurred during the various test phases:
- controls, if any, are valid;
- samples respect the acceptance criteria.

The control validity criteria and sample acceptance criteria of the various phases are specified above.

### Assessment of results - Analysis of results

Result analysis consisted of assessing the species identified by the MAGNETO bioinformatics pipeline. The report produced by the pipeline contained a table for the species identified in the sample as follows:
- hamster: hamster species has been identified
- human: human species has been identified
- mouse: murine species has been identified

The report may therefore contain 1, 2 or 3 tables depending on the species identified.

### Result compliance

The test result for a certain sample was deemed compliant when:
- the cell line species of origin was confirmed
- there was no contamination by cell lines of other species

The report produced by the MAGNETO pipeline on sample analysis should therefore contain only the table for the species of origin of that sample.

**Table 3: Examplary profile of identification.**

| **Gene** | **SNP No.** | **Name of the SNP** | **Hamster** | **Mouse** | **Human** |
|---|---|---|---|---|---|
| **Ago1** | **1** | | A | T | C |
| | **2** | | T | T | A |
| | **3** | | C | T | C |
| **Cytb** | **4** | | A | A | T |
| | **5** | | | A | |
| **Hdac1** | **6** | | C | C | T |
| | **7** | | T | G | C |
| | **8** | | G | T | A |
| | **9** | | C | C | G |
| | **10** | | A | C | T |
| **Srsf1** | **11** | | C | C | T |
| | **12** | | C | C | T |
| | **13** | | A | A | G |
| | | | | | |

| **Gene** | **SNP No.** | **Name of the SNP** | **Hamster** | **Mouse** | **Human** |
|---|---|---|---|---|---|
| **Top2b** | **14** | | C | C | T |
| | **15** | | G | G | A |
| | **16** | | G | A | G |
| | **17** | | C | T | A |
| | **18** | | C | T | C |
| | **19** | | C | T | T |
| | **20** | | G | A | T |
| | **21** | | C | C | T |
| | **22** | | A | G | A |

### Example 1 - Validation tests

During validation, tests were carried out with the aim of checking the ability of the method to identify cell lines of various species. In particular, specificity was proven checking:
1. the ability of the method to confirm the species of origin of a human, murine or hamster cell line,
2. the ability of the method to confirm the species of origin of a cell bank previously tested using the "Isoenzyme analysis in cell lines" method.

In the first test (i.e. ability of the method to confirm the species of origin of a human, murine or hamster cell line), specificity was assessed using the following samples:
- CHO-KI,
- MRC-5,
- Sp2-0/Ag14.

Below are the results of the specificity check giving the species identified for each sample

| Sample | Species identified | | |
|---|---|---|---|
| | Expected results | Assay 1 | Assay 2 |
| CHO-K1 | Hamster | Hamster | Hamster |
| MRC-5 | Human | Human | Human |
| Sp2-0/Ag14 | Mouse | Mouse | Mouse |

In the second test (i.e. ability of the method to confirm the species of origin of a cell bank previously tested using the "Isoenzyme analysis in cell lines" method), specificity was assessed using the following samples:
- mAb1 cell (Sp2-0/Ag14),
- mAb2 cell (CHO-S).

Below are the results of the specificity check giving the species identified for each sample.

| Sample | Species identified | |
|---|---|---|
| | Expected results | Essay 1 |
| mAb1 cell (Sp2-0/Ag14) | Mouse | Mouse |
| mAb2 cell (CHO-S) | Hamster | Hamster |

All the results obtained in both tests for checking method specificity are valid according to the acceptance criteria (see main methods section), and comply with the acceptance criterion, since the species of origin was confirmed for all the samples tested. Based on these results, it can be stated that the method is specific.

### Example 2 - Cross contamination tests

Tests were carried out with the aim of checking the limit of detection, intended as the lowest percentage of contamination among the test species (man, mouse and hamster) that the method is able to identify. To check the limit of detection, cell pellets were prepared mixing different percentages of human (MRC-5), mouse (SP2/0-Ag14) and hamster (CHO-K1) cells lines, as shown below (Table 4).

**Table 4**

| | Contaminating species | | | | | | | | |
|---|---|---|---|---|---|---|---|---|---|
| | SP2/0 | | | MRC-5 | | | CHO-K1 | | |
| SP2/0-Ag14 | - | - | - | 0.5% | 5% | 10% | 0.5% | 5% | 10% |
| MRC-5 | 0.5% | 5% | 10% | - | - | - | 0.5% | 5% | 10% |
| CHO-K1 | 0.5% | 5% | 10% | 0.5% | 5% | 10% | - | - | - |

The samples were prepared, sequenced on a Micro flow cell and analyzed along with samples for checking specificity, as per main methods section. Three repetitions of the test were done using 3 different cell pellets of each mix.

**Table 5**

| Cell line present at highest % | Contaminating species | Identification of contaminating species | | | | | | | | |
|---|---|---|---|---|---|---|---|---|---|---|
| | | Assay 1 | | | Assay 2 | | | Assay 3 | | |
| | | 0.5% | 5% | 10% | 0.5% | 5% | 10% | 0.5% | 5% | 10% |
| SP2/0-Ag 14 | MRC-5 | No | Yes | Yes | No | Yes | Yes | No | Yes | Yes |
| | CHO-K1 | No | Yes | Yes | Yes | Yes | Yes | No | Yes | Yes |
| MRC-5 | SP2/0-Ag 14 | No | Yes | Yes | No | Yes | Yes | No | Yes | Yes |
| | CHO-K1 | No | Yes | Yes | No | Yes | Yes | Yes | Yes | Yes |
| CHO-K1 | SP2/0-Ag 14 | No | Yes | Yes | Yes | Yes | Yes | No | Yes | Yes |
| | MRC-5 | No | Yes | Yes | No | Yes | Yes | No | Yes | Yes |

In the mixes containing 5% or more of contaminating species, the contaminating species was identified in 3 repetitions out of 3 of the test (100% success). Based on these results it can therefore be concluded that the method is able to detect cross-contamination among the cell lines of the 3 different test species (man, mouse and hamster) with a 5% minimum limit of contamination detectable (LOD).

### Example 3 - Robustness tests

Robustness, intended as the ability of the method to remain unchanged in spite of the introduction of deliberate changes in some parameters considered critical, was assessed.

In particular, each experimental phase of the method was subjected to risk analysis in order to identify critical phases for robustness checking.

The experimental phases found to be critical were:
1. PCR and analysis of the result by electrophoretic run on agarose gel and image analysis,
2. Library preparation,
3. Library loading and running on MiSeq sequencer.

As regards the genomic DNA amplification phase, the PCR (quantity of Taq polymerase enzyme in the reaction) and visualization of bands obtained on agarose gel (different intercalators of DNA in the gel and gel acquisition and analysis using different instruments) conditions were varied.

In the library preparation phase, the quantity of starting DNA was changed, as was the quantity of tagmentation enzyme needed to fragment the DNA.

Finally, as regards the library loading phase and run on the MiSeq sequencer, a check was done on robustness of the method in sequencing samples consisting of recombinant cell banks loaded on a Nano flow cell.

Robustness of this phase was also checked during method validation by assessing the results from samples loaded on two different types of flow cell for sequencing, Nano or Micro. In particular, robustness was proven by checking:
1. the ability of the method to confirm the species of origin of a cell line loaded and sequenced on a Nano or on a Micro flow cell.
2. the ability of the method to identify the contaminating species present at the LOD in samples of murine or hamster origin loaded and sequenced on a Nano or on a Micro flow cell.
1. Robustness check intended as the ability of the method to confirm the species of origin of a cell line loaded and sequenced on a Nano or on a Micro flow cell. Robustness was assessed on the results from samples prepared and analyzed for the method specificity check (example 1). The samples used were:
- CHO-K1, MRC-5 and Sp2-0/Ag14, loaded and sequenced on a Micro flow cell,
- mAb1 cells and mAb2 cells, loaded and sequenced on a Nano flow cell.
The acceptance criterion for checking robustness was to have confirmation of the species of origin of the samples loaded and sequenced both on the Nano and on the Micro flow cell. Below are the results of samples loaded and sequenced on a Micro flow cell:

| Sample | Species identified (sequencing on a micro flow cell) | | |
|---|---|---|---|
| | Expected results | Assay 1 | Assay 2 |
| CHO-K1 | Hamster | Hamster | Hamster |
| MRC-5 | Human | Human | Human |
| Sp2-0/Ag14 | Mouse | Mouse | Mouse |

Below are the results of samples loaded and sequenced on a Nano flow cell:

| Sample | Species identified (sequencing on a micro flow cell) | |
|---|---|---|
| | Expected results | Essay 1 |
| mAb1 cell (Sp2-0/Ag14) | Mouse | Mouse |
| mAb2 cell (CHO-S) | Hamster | Hamster |

2. Robustness check intended as the ability of the method to identify the contaminating species present at the LOD in samples of murine and of hamster origin loaded and sequenced on a Nano or on a Micro flow cell. Robustness was assessed using the following samples:
- Mix consisting of 95% of CHO-K1 cells and 5% (LOD) of Sp2-0/ Ag14 cells
- Mix consisting of 95% of Sp2-0/ Ag14 cells and 5% (LOD) of CHO-K1 cells

Robustness of the method in identifying the contaminating species present at the LOD in samples loaded and sequenced on a Micro flow cell was assessed on the results of assay 3 of the test for method limit of detection check (see example 2). Robustness of the method in identifying the contaminating species present at the LOD in samples loaded and sequenced on a Nano flow cell was assessed using the same samples. In particular, the libraries of samples of the CHO-K1 cell line mixed at the LOD with Sp2-0/ Ag14 and of the Sp2-0/Ag14 cell line mixed at the LOD with CHO-K1 of the third repetition of the limit of detection test were loaded and sequenced on a Nano flow cell.
Below are the results from samples loaded and sequenced on the Micro flow cell

| Sample | Species of origin obtained | Contaminating species identified |
|---|---|---|
| 95% CHO-K1 + 5% Sp2-0/Ag14 | Hamster | Mouse |
| 95% Sp2-0/Ag14 + 5% CHO-K1 | Mouse | Hamster |

Below are the results from samples loaded and sequenced on the Nano flow cell

| Sample | Species of origin obtained | Contaminating species identified |
|---|---|---|
| 95% CHO-K1 + 5% Sp2-0/Ag14 | Hamster | Mouse |
| 95% Sp2-0/Ag14 + 5% CHO-K1 | Mouse | Hamster |

All the results of both method robustness checking tests (point 1 and point 2) are valid according to the acceptance criteria, and comply with these criteria. The method can therefore be considered as being robust with respect to the parameters considered critical for its performance.

The method was classified as a "Limit test for impurities". The parameters validated were specificity, limit of detection (LOD) and robustness.
- Validation results show that that method is SPECIFIC.
- The method is able to detect cross-contamination among cell lines of the 3 different test species (man, mouse and hamster) with a 5% minimum limit of contamination detectable (LOD).
- Moreover, the method was found to be ROBUST with respect to the parameters considered critical for its performance.
Consequently, the "Mammalian cell line identity by Next Generation Sequencing" method used to confirm the species of origin of cell lines and to assess any cross-contamination with cells of other species, is to be considered VALIDATED. It can efficiently replace the typical Isoenzyme analysis routinely used for confirm the species of origin of cell lines.

## Claims

1. A method for identifying the specific cell lineage of cells in culture comprising the steps of: 1) determining from the nucleic acid molecules isolated from said recombinant cells in culture the presence of polymorphisms or SNPs at at least 20 different positions within at least five genes contained in said nucleic acid molecules, 2) obtaining a genetic profile from the determination of step 1), and 3) identiyfing the cell lineage of said cells in culture from said genetic profile;
wherein the at least five genes are: Argonaute RISC catalytic component 1 (Ago1), Cytochrome b (Cytb), Histone deacetylase 1 (Hdac1), Serine/arginine-rich splicing factor 1 (Srsf1) and Topoisomerase II beta (Top2b), and wherein the recombinant cells produce a recombinant protein.

2. An analytic method comprising the steps of: 1) analyzing the nucleic acid molecules isolated from recombinant cells in culture to determine the presence of polymorphisms or SNPs at at least 20 different positions within at least five genes contained in said nucleic acid molecules, 2) obtaining a genetic profil from the analysis of step 1), and 3) determining the species of said recombinant cells in culture from said genetic profile;
wherein the at least five genes are: Argonaute RISC catalytic component 1 (Ago1), Cytochrome b (Cytb), Histone deacetylase 1 (Hdac1), Serine/arginine-rich splicing factor 1 (Srsf1) and Topoisomerase II beta (Top2b), and wherein the recombinant cells produce a recombinant protein.

3. A method for cell bank characterisation of recombinant cells in culture comprising the steps of: 1) determining, in nucleic acid molecules isolated from said recombinant cells in culture, the presence of polymorphisms or SNPs at at least 20 different positions within at least five genes, 2) obtaining a genetic profile from the detection of step 1), and 3) characterizing the origine of the cell bank of the recombinant cells in culture from said genetic profile;
wherein the at least five genes are: Argonaute RISC catalytic component 1 (Ago1), Cytochrome b (Cytb), Histone deacetylase 1 (Hdac1), Serine/arginine-rich splicing factor 1 (Srsf1) and Topoisomerase II beta (Top2b), and wherein the recombinant cells produce a recombinant protein.

4. The method according to any one of the preceding claims wherein the genetic profile correlates with cell lineage, species and/or origine of the cell bank of said recombinant cells in culture.

5. The method according to any one of the preceding claims, wherein step 3) is made by comparing said genetic profile to a reference genetic profile, wherein the comparison is indicative of the cell lineage, species and/or origine of the cell bank of said recombinant cells in culture.

6. The method according to any one of the preceding claims wherein the presence of polymorphsims or SNPs at said at least 20 different positions in the at least five genes is determined according to a method that includes the steps of: a) isolating a sample of recombinant cells from the cell in culture, 2) isolating the nucleic acid molecules from the cells isolated in step 1), 3) hybridizing specific pairs of primers to the nucleic acid molecules comprising the at least five genes of interest; 4) amplifying said nucleic acid molecules to obtaine amplified nucleic acid molecule fragments, and 5) sequencing said nucleic acid molecule fragments.

7. The method according to claim 6 wherein the specific pair of primers generate paired-end sequences allowing sequencing of each given nucleic acid molecule from both ends of said nucleic acid molecules, thereby generating pairs of reads for each given nucleic acid molecule representing one of the genes of interest.

8. The method according to any one of the preceding claims wherein the sequencing of said nucleic acid is made by Sanger method, or Next Generation Sequencing (NGS)

9. The method according to any one of the preceding claims wherein the recombinant protein is selected from the group consisting of an antibody or antigen binding fragment thereof, such as a human antibody or antigen-binding portion thereof, a humanized antibody or antigen-binding portion thereof, a chimeric antibody or antigen-binding portion thereof, a recombinant fusion protein, a growth factor, a hormone, or a cytokine.

10. An apparatus for determining the genetic profile of recombinant cells in culture, comprising: 1) a computer readable memory; 2) a computer programme stored on the computer readable memory adapted to be executed on a processor to analyze the genetic sequencing data obtained from said cells with regard to the polymorphisms or SNPs at at least 20 different positions within at least five genes contained said cells and 3) generate the genetic profile as an output based on said polymorphisms or SNPs, wherein the at least five genes are: Argonaute RISC catalytic component 1 (Ago1), Cytochrome b (Cytb), Histone deacetylase 1 (Hdac1), Serine/arginine-rich splicing factor 1 (Srsf1) and Topoisomerase II beta (Top2b), and wherein the recombinant cells produce a recombinant protein.

11. The apparatus according to claim 10, further comprising the step 4) of comparing said genetic profile to a reference genetic profile, stored in the computer readable memory, wherein the comparison is indicative of the cell lineage, species and/or origine of the cell bank of said recombinant cells in culture.

12. A system comprising the apparatus according to any one of claims 10 or 11 and further comprising a display operably coupled to the processor to display the output.

13. The system according to claim 12, further comprising a database operatively connected to the processor and adapted to store information about the genetic profile of recombinant cells, said information including the identity of the polymorphisms or SNPs at said at least 20 different positions within said at least five genes.

14. The system according to claim 13, wherein the database further includes the cell lineage, species and/or origine of the cell bank of said recombinant cells in culture, as a result of the obtention of the genetic profile.

15. A kit for amplification of at least 20 polymorphisms or SNPs sites in at least five genes located in nucleic acid molecules isolated from recombinant cells in culture, the kit comprising at least 20 pairs of primers which hybridize with said nucleic acid molecules at at least 20 polymorphisms or SNPs sites, wherein the at least five genes are: Argonaute RISC catalytic component 1 (Ago1), Cytochrome b (Cytb), Histone deacetylase 1 (Hdac1), Serine/arginine-rich splicing factor 1 (Srsf1) and Topoisomerase II beta (Top2b), and wherein the recombinant cells produce a recombinant protein.

16. The kit according to claim 15, wherein the pairs of primers are capable to hybridize and amplify said at least 20 polymorphisms or SNPs sites.

17. The kit according to claims 15 or 16 further comprising the reagents and oligonucleotides necessary to amplify the regions to which the pairs of primers have hybridized.
